(19) Europäisches Patentamt
European Patent Office
Office européen des brevets

(11) Publication number: **0 427 529 B1**

(12) **EUROPEAN PATENT SPECIFICATION**

(45) Date of publication of patent specification: **19.04.95**  (51) Int. Cl.6: **A01N 65/00**

(21) Application number: **90312171.3**

(22) Date of filing: **07.11.90**

(54) **Larvicidal lectins and plant insect resistance based thereon.**

(30) Priority: **07.11.89 US 433625**

(43) Date of publication of application:
**15.05.91 Bulletin  91/20**

(45) Publication of the grant of the patent:
**19.04.95 Bulletin  95/16**

(84) Designated Contracting States:
**AT BE CH DE DK ES FR GB GR IT LI LU NL SE**

(56) References cited:
**EP-A- 0 204 590**
**EP-A- 0 351 924**

(73) Proprietor: **PIONEER HI-BRED INTERNATIONAL, INC.**
**700 Capital Square**
**400 Locust Street**
**Des Moines**
**Iowa 50309 (US)**

(72) Inventor: **Cavalieri, Anthony**
**8498 NW Beaver,**
**Johnston**
**Polk County,**
**Iowa 50131 (US)**
Inventor: **Czapla, Thomas**
**6129 Meadowcrest Drive,**
**Apt. 205,**
**Johnston**
**Polk County,**
**Iowa 50131 (US)**
Inventor: **Howard, John**
**3211 Valley Ridge Court,**
**West Des Moines**
**Polk County,**
**Iowa 50265 (US)**

(74) Representative: **Goldin, Douglas Michael et al**
**J.A. KEMP & CO.**
**14, South Square**
**Gray's Inn**
**London WC1R 5LX (GB)**

EP 0 427 529 B1

## Description

Technical Field

This invention relates to materials and methods for killing insect larvae which are harmful to plants, and materials and methods for imparting insect resistance to plants.

Background of the Invention

Numerous insects are serious pests of common agricultural crops. One method of controlling insects has been to apply insecticidal organic or semiorganic chemicals to crops. This method has numerous, art-recognized problems. A more recent method of control of insect pests has been the use of biological control organisms which are typically natural predators of the troublesome insects. These include other insects, fungi (milky-spore) and bacteria (Bacillus thuringiensis cv.). However, it is difficult to apply biological control organisms to large areas, and even more difficult to cause those living organisms to remain in the treated area for an extended period. Still more recently, techniques in recombinant DNA have provided the opportunity to insert into plant cells cloned genes which express insecticidal toxins derived from biological control organisms such as Bt. This technology has given rise to additional concerns about eventual insect resistance to well-known, naturally occurring insect toxins, particularly in the face of heavy selection pressure, which may occur in some areas. Thus, a continuing need exists to identify naturally occurring insecticidal toxins which can be formed by plant cells directly by translation of a single structural gene.

European Patent Application 204,590, based upon U.S. Patent application Serial No. 725,368, describes a method of genetically modifying a plant cell to control expression of heterologous foreign structure genes, including a lectin from Phaseolus vulgaris. In the method, the plant cell is transformed to contain a pRi T-DNA promoter and a heterologous foreign structural gene, the promoter and the structural gene being in such position and orientation with respect to one another that the structural gene is expressible in a plant cell under control of the promoter.

Likewise, European Patent Application 186,425, based upon U.S. patent application Serial No. 685,824, describes a recombinant DNA expression vector which comprises (a) a transcription unit, flanked by T-DNA border sequences, which comprises a promoter and associated amino terminal region encoding sequences and a terminator signal sequence in which the sequences are derived from one or more genes which are naturally expressed in a plant cell, and (b) an antibiotic resistance gene-encoding sequence located between the promoter and associated amino-terminal region-encoding sequence and the terminator sequence and (c) a DNA fragment containing a replicon that is functional in Agrobacterium.

PCT application 8807087, based upon U.S. patent application 168,109, discloses a recombinant virus expression system comprising a Heliothis polyhedrin promoter and a nucleotide sequence encoding a heterologous peptide or protein, which may have insecticidal activity.

On the other hand, at least one of the lectins employed herein is taught to be produced in an insect cell/baculovirus expression system, as disclosed in PCT patent application 89-01037, based upon U.S. Patent Application Serial Number 153778, which makes its potent insecticidal activity surprising.

European Patent Application 237,676, based on U.S. application 837,583, describes a recombinant DNA sequence which codes for any of a) ricin A chain protein; b) the B chain portion of the ricin precursor protein; c) the A chain and B chain portions of the ricin precursor protein; or d) ricin precursor protein or polypeptide. An expression system is described as including one of the foregoing DNA sequences operably linked to a control sequence compatible with a recombinant host cell. The objective is to produce ricin toxin, precursor or fragments thereof in commercial quantities.

European Patent Application 204,590, based on U.S. patent application 725,368, relates to a method of genetically modifying a plant cell with a pRi tDNA promoter and a heterologous foreign structural gene, such as the lectin from Phaseolus vulgaris. The heterologous foreign structural gene is in position and orientation with respect to the promoter such that the structural gene is expressible in a plant cell under the control of the promoter. Expression of the Phaseolus vulgaris lectin is stated to be useful in improving the nutritional quality of the plant cell proteins.

Brief Description of the Drawings

Figure 1 illustrates the gene map of plasmid pPHI414 which is useful as an expression cassette for lectin structural genes.

2

Figure 2 illustrates the gene map of plasmid pPHI412 which is also useful as an expression cassette for lectin structural genes.

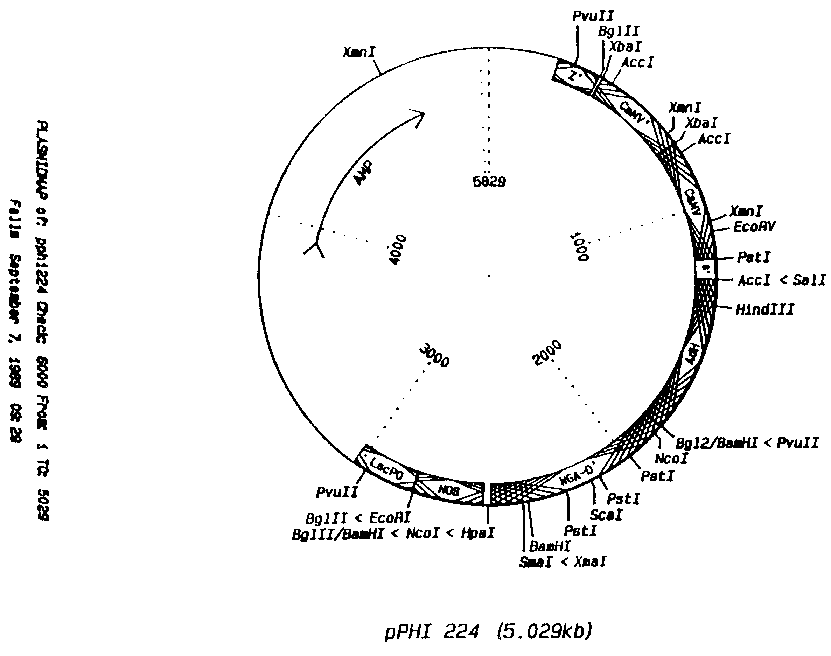

Figure 3 illustrates the gene map of plasmid pPHI224 which is a specific expression cassette for containing the structural gene for the lectin Wheat Germ Agglutinin.

## Disclosure of the Invention

It has now been determined that certain lectins have potent larvicidal activity when administered orally to the larvae of European corn borer, corn rootworm, and cutworm. Thus, this invention provides a method for killing or inhibiting the growth of insect larvae selected from European corn borer, corn rootworm and cutworm, comprising administering orally to the larvae a larvicidal amount of a lectin selected from Artocarpus integrifolia lectin (jacalin), Bauhinia purpurea alba (camel's foot tree) lectin (BPA), Codium fragile lectin (CFL), elderberry bark lectin (EBL), Griffonia simplicifolia (GSL), Phytolacca americana lectin (PAL), Maclura pomifera (osage orange) lectin (MPL), Ricinus communis (castor bean) agglutinin (RCA), Triticum vulgare lectin (Wheat germ agglutinin, WGA), Vicia villosa lectin (VVL) and combinations thereof. As used herein, larvicidal amount means at least an amount sufficient to cause a 50% inhibition of weight gain in treated larvae ($ED_{50}$) and/or mortality in 25% of treated larvae ($LD_{25}$). The lectin can be effectively applied to plants consumed by the larvae by spray, dust or other formulation common to the insecticidal arts. Alternatively, the lectin can be incorporated into the tissues of a susceptible plant so that in the course of infesting the plant the larvae consume larvicidal amounts of the selected lectin or lectins. One method of doing this is to incorporate the lectin in a non-phytotoxic vehicle which is adapted for systemic administration to the susceptible plants. This method is commonly employed with insecticidal materials which are designed to attack chewing insects and is well within the purview of one of ordinary skill in the art of insecticide and larvicide formulation. However, since the genes which code for these lectins can be isolated, cloned, inserted into an appropriate expression cassette, and introduced into cells of a susceptible plant species, an especially preferred embodiment of this method involves inserting into the genome of the plant a DNA sequence coding for an insecticidal plant lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Triticum vulgare lectin (Wheat germ agglutinin), and Vicia villosa lectin in proper reading frame relative to transcription initiator and promoter sequences active in the plant. Transcription and translation of the DNA sequence under control of the regulatory sequences causes expression of the lectin protein sequence at levels which provide an insecticidal amount of the lectin in the tissues of the plant which are normally infested by the larvae. Alternatively, a dietary bait containing the selected lectin or combination of lectins can be employed, with, optionally, an added hormonal or other larval attractant material.

The plant is preferably a plant susceptible to infestation and damage by the larvae of one or more of European corn borer, corn rootworm and cutworm. These include corn (Zea mays) and sorghum (Sorghum bicolor). However, this is not to be construed as limiting, inasmuch as these species are among the most difficult commercial crops to reliably transform and regenerate, and these insects (under other common names) also infest certain other crops. Thus the methods of this invention are believed to be readily applicable via conventional techniques to numerous plant species, if they are found to be susceptible to the plant pests listed hereinabove, including, without limitation, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersionn, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum, and Datura.

Preferred plants that are to be transformed according to the methods of this invention are cereal crops, including maize, rye, barley, wheat, sorghum, oats, millet, rice, triticale, sunflower, alfalfa, rape seed and soybean.

The DNA sequence which when expressed imparts insecticidal activity is a structural gene which codes for one of the selected plant lectins described herein i.e., jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Triticum vulgare lectin (Wheat germ agglutinin), and Vicia villosa lectin. It has been found that, although numerous lectins are known to biological science and a few of these have now been found to exhibit some insecticidal activity, only those described herein have been found to have sufficient insecticidal (larvicidal) activity to be operative in a plant cell expression system. That is, while other lectins have some larvicidal activity at high concentrations in pure form, plant cell expression at such high concentrations is either not likely in a living plant cell system, or is not likely while preserving the commercially useful characteristics of the plant in terms of production of oils, starches, fibers, or other materials. In addition, lectins which are active against some insects are inactive against others. For example, wheat germ agglutinin, one of the preferred lectins herein, is very toxic to the larvae of the European corn borer but relatively nontoxic to tobacco budworm and Southern Corn Rootworm. Soybean lectin, Con A and peanut lectin have not shown significant activity against any insects tested to date.

In general, since the object of the invention is to confer resistance to an insect to which the plant is susceptible, the selected lectin will not be native to the plant, i.e., the lectin will come from a species other than the plant being transformed. However, in species which produce larvicidal lectins but in lower than larvicidal amounts, it may be preferable to insert a gene for the native lectin under strong constitutive promoter control to cause overproduction of the lectin, thus achieving larvicidal levels and conferring effective insect resistance. Alternatively, where a plant produces a native larvicidal lectin but the lectin is not produced in or not distributed to tissues which are normally infested by the larvae, a tissue specific promoter can be used to provide localized expression or overproduction of the lectin. A tissue specific promoter can be used in any instance where it may be desirable to localize production of the lectin to an infested tissue or to a tissue which is efficient in production of the lectin.

The DNA sequences which code for the lectins of this invention can be obtained by conventional techniques. Ricinus communis agglutinin, for example, has been sequenced to the extent that DNA probes can be constructed to locate the native gene in the Ricinus communis genome, and the gene can then be removed by use of appropriate restriction enzymes and spliced into a selected plant expression cassette. Alternatively, the lectin can be sequenced in its entirety using known methods, and synthetic DNA sequences can then be prepared which code for the appropriate sequence of amino acids, and this synthetic sequence can be inserted into an appropriate plant expression cassette. These techniques are applicable to each of the lectins identified herein as useful in this invention.

Likewise, numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong constitutive promoter sequence at one end to cause the gene to be transcribed at a high frequency, and a poly-A recognition sequence at the other end for proper processing and transport of the messenger RNA. An example of such a preferred (empty) expression cassette into which the cDNA of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endonuclease recognition sites which may be cut in a predictable fashion by use of the appropriate enzyme. Such vectors are preferably constructed to include additional structural gene sequences imparting antibiotic or herbicide resistance, which then serve as markers to identify and separate transformed cells. Preferred markers/selection agents include kanamycin, chlorosulfuron, phosphonothricin, hygromycin and methotrexate. A cell in which the foreign genetic material in a vector is functionally expressed has been "transformed" by the vector and is referred to as a "transformant."

A particularly preferred vector is a plasmid, by which is meant a circular double-stranded DNA molecule which is not a part of the chromosomes of the cell.

As mentioned above, both genomic and cDNA encoding the gene of interest may be used in this invention. The vector of interest may also be constructed partially from a cDNA clone and partially from a genomic clone. When the gene of interest has been isolated, genetic constructs are made which contain the necessary regulatory sequences to provide for efficient expression of the gene in the host cell. According to this invention, the genetic construct will contain (a) a first genetic sequence coding for the protein or trait of interest and (b) one or more regulatory sequences operably linked on either side of the structural gene of interest. Typically, the regulatory sequences will be selected from the group comprising promoters and terminators. The regulatory sequences may be from autologous or heterologous sources.

Promoters that may be used in the genetic sequence include nos, ocs and CaMV promoters.

An efficient plant promoter that may be used is an overproducing plant promoter. Overproducing plant promoters that may be used in this invention include the promoter of the small sub-unit (ss) of the ribulose-1,5-biphosphate carboxylase from soybean (Berry-Lowe et al, J. Molecular and App. Gen., 1:483-498 (1982)), and the promoter of the cholorophyll a-b binding protein. These two promoters are known to be light-induced, in eukaryotic plant cells (see, for example, Genetic Engineering of Plants, An Agricultural Perspective, A. Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)).

The expression cassette comprising the structural gene for the lectin of interest operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as E. Coli, S. typhimurium, and Serratia marcescens. Eukaryotic hosts such as yeast or filamentous fungi may also be used in this invention.

The isolated cloning vector will then be introduced into the plant cell using any convenient technique, including electroporation (in protoplasts), retroviruses, bombardment, and microinjection, into cells from monocotyledonous or dicotyledonous plants, in cell or tissue culture, to provide transformed plant cells containing as foreign DNA at least one copy of the DNA sequence of the plant expression cassette. Preferably, the monocotyledonous species will be selected from maize, sorghum, wheat and rice, and the dicotyledonous species will be selected from soybean, alfalfa, tobacco and tomato. Using known techniques, protoplasts can be regenerated and cell or tissue culture can be regenerated to form whole fertile plants which carry and express the desired gene for the selected lectin. Accordingly, a highly preferred embodiment of the present invention is a transformed maize plant, the cells of which contain as foreign DNA at least one copy of the DNA sequence of an expression cassette of this invention.

Finally, this invention provides methods of imparting resistance to insects selected from European corn borer, corn rootworm, and cutworm to plants of a susceptible taxon, comprising the steps of:

a) culturing cells or tissues from at least one plant from the taxon,

b) introducing into the cells of the cell or tissue culture at least one copy of an expression cassette comprising a structural gene for a lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Triticum vulgare lectin (Wheat germ agglutinin), and Vicia villosa lectin, operably linked to plant regulatory sequences which cause the expression of the lectin structural gene in the cells, and

c) regenerating insect-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the lectin structural gene and associated regulatory sequences via crossing and backcrossing. Such intermediate methods will comprise the further steps of

a) sexually crossing the insect-resistant plant with a plant from the insect-susceptible taxon;

b) recovering reproductive material from the progeny of the cross; and

c) growing insect-resistant plants from the reproductive material. Where desirable or necessary, the agronomic characteristics of the susceptible taxon can be substantially preserved by expanding this method to include the further steps of repetitively:

a) backcrossing the insect-resistant progeny with insect-susceptible plants from the susceptible taxon; and

b) selecting for expression of insect resistance (or an associated marker gene) among the progeny of the backcross, until the desired percentage of the characteristics of the susceptible taxon are present in the progeny along with the gene imparting insect resistance.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incorporated into Agrobacterium tumefaciens, which can then be used to transfer the vector into susceptible plant cells, primarily from dicotyledonous species. Thus, this invention provides a method for imparting insect resistance in Agrobacterium tumefaciens-susceptible dicotyledonous plants in which the expression cassette is introduced into the cells by infecting the cells with Agrobacterium tumefaciens, a plasmid of which has been modified to include a plant expression cassette of this invention.

The following description further exemplifies the compositions of this invention and the methods of making and using them. However, it will be understood that other methods, known by those of ordinary skill in the art to be equivalent, can also be employed.

### Example 1

### Insect Susceptibility to Lectins

Since one of the primary routes of administration of insecticidal substances to insect pests is via the digestive tract, the susceptibility of selected insect larvae to an assortment of lectins administered in the diet was evaluated.

European cornborers (ECB), Ostrinia nubilalis (Hubner), Southern corn rootworm (SCR), Diabrotica undecimpunctata howardi Barber, were used to screen lectins for anti-insect activity. In all bioassays, ECB and SCR larvae were reared in tissue culture tray cells (4 x 6 cells/tray) that contained approximately 1.5 g of Stoneville artificial media (pH 5.6). Three treatments plus one control, each composed of one row of six cells, were infested with 2 neonate larvae per cell. A mylar film was adhered to the top of each tray after infestation to prevent escape of the larvae. The mylar was then punctured with insect pins (size 0) and attached to a cork stopper to provide adequate air flow.

### Bioassays of Insect Susceptibility to Orally Administered Lectins

A 2% solution of each lectin (Ricinus communis agglutinin was obtained as a 0.5% solution) was prepared in 0.1 M PBS buffer (pH 7.8). Seventy-five $\mu$l were then applied topically to the surface of the media in each cell. After the solution had dried, the cell was infested with two neonate larvae. The control treatment consisted of a 75 $\mu$l application of buffer only per cell. Larval weights and mortalities were recorded after seven days and compared to those of the control treatment. Lectins that inhibited larval growth by at least 50% or produced at least 25% mortality when compared to the control treatment were tested again. Lectins that continued to display anti-insect activity were then tested using a bioassay in which the lectin was incorporated in the diet.

### RESULTS

Results are shown in Tables 1 and 2. Forty-three lectins, differing in carbohydrate-binding specificity, were examined for anti-insect activity against neonate ECB and SCR larvae by these bioassays. Ten lectins showed positive results, i.e. caused at least either 25% mortality and/or a 50% weight reduction in treated larvae compared to control larvae. These ten lectins were Artocarpus integrifolia lectin (jacalin), Bauhinia purpurea alba (BPA) lectin, Codium fragile lectin (CFL), Elderberry bark lectin (EBL), Griffonia simplicifolia - (GSII), Phytolacca americana lectin (PAL), Maclura pomifera lectin (MPL), Ricinus communis agglutinin (RCA), Triticum vulgare lectin (Wheat germ agglutinin, WGA), and Vicia villosa lectin (VVL). Three of these materials were active against ECB larvae, while nine had activity against SCR larvae.

RCA, WGA, and BPA were the only lectins of the 46 tested which displayed antilarval effectiveness on neonate ECB larvae. At the 2% test level of lectin applied topically to the medium, 100% mortality occurred within seven days in all treated cells (Table 1). WGA and RCA maintained their anti-insect activity down to the 0.1% level by inhibiting weight gain by 50% (data not shown). None of the other lectins tested had anti-insect activity against ECB as determined by the preset levels. The addition of 2% casein topically to the media surface did not affect larval weights, indicating that other proteins do not interfere with the insecticidal activity of the lectin.

Lectins from a variety of plant and animal sources as indicated below were screened for anti-insect activity against neonate European Cornborer and Southern Corn rootworm larvae[1].

| Species | M.W. (Kd) | Anti-Insect |
|---|---|---|
| Artocarpus integrifolia | 40 | Yes |
| Arachis hypogaea | 110 | No |
| Bauhinia purpurea alba | 195 | Yes |
| Canavalia ensiformis | 104 | No |
| Codium fragile | 60 | Yes |
| Datura stramonium | 86 | No |
| Dolichos biflorus | 120 | No |
| Erythrina cristagalli | 54 | No |
| Elderberry Bark Lectin | 150 | Yes |
| Glycine max | 120 | No |
| Griffonia simplicifolia: | | |
| I | 115 | No |
| II | 115 | Yes |
| Lens culinaris | 49 | No |
| Limulus polyphemus | 400 | No |
| Lotus tetrabonolobus | 56–112 | No |
| Lycopersicon esculentum | 100 | No |
| Maclura pomifera | 40–43 | Yes |
| Naja mocambique | | No |
| Perseau americana | | No |
| Phytolacca americana | 32 | Yes |
| Pisum sativum | 46 | No |
| Phaseolus cocineus | 112 | No |
| Phaseolus limensis | 247(II) | No |
| | 247(III) | |
| Phaseolus vulgaris E | 125 | No |
| Phaseolus vulgaris L | 125 | NO |
| Ricinus communis | 120 | Yes |
| Sambucus nigra | 140 | No |

| | | |
|---|---|---|
| Salanum tuberosum | 100 | No |
| Sophora japonica | 133 | No |
| Triticum vulgare | 36 | Yes |
| Ulex europaeus I | 170 | No |
| Ulex europaeus II | 170 | No |
| Vicia villosa | 139 | Yes |
| Viscum album | 115 | No |
| Wistaria floribunda | 68 | No |
| | 136 | Yes |
| Semi-purified Lectins | | |
| Sopharo Leaf doublet | | No |
| Sopharo Bark Lectin II | | No |
| Sopharo Bark Lectin III | | No |
| Sopharo Bark Lectin V | | No |
| Sopharo Leaf Lectin I | | No |
| Sopharo Leaf Lectin II | | No |
| Phaseolus Tepary | | No |
| Sparticum Spanish bloom | | No |

[1]Anti-Insect activity was predetermined to be 25% mortality and/or 50% weight loss after a 7-day, 2% topical application to the medium for either insect when compared to the control larvae.

## Table 2

Effect of anti-insect lectins topically applied to medium on mortality and weight of neonate European corn borer and Southern corn rootworm larvae[1]

| Lectin | Insect[2] | Weight[3] | % Weight[4] Loss | %[5] Mortality | n[6] |
|---|---|---|---|---|---|
| Specificity: N-acetylglucosamine | | | | | |
| GSL II | ECB | 5.0 ± 0.4 | 15 | 0 | 6 |
| | SCR | 0.3 ± 0.1* | 80 | 15 | 12 |
| PAL | ECB | 5.8 ± 0.6 | 0 | 0 | 6 |
| | SCR | 1.4 ± 0.4 | 0 | 35 | 12 |
| WGA | ECB | N.A. | N.A. | 100 | 18 |
| | SCR | 0.6 ± 0.1* | 58 | 10 | 36 |
| Specificity: N-acetylgalactosamine/galactose | | | | | |
| jacalin | ECB | 5.8 ± 0.6 | 0 | 0 | 6 |
| | SCR | 0.4 ± 0.1* | 70 | 0 | 36 |
| BPA lectin | ECB | N.A. | N.A. | 100 | 12 |
| | SCR | 0.9 ± 0.2* | 35 | 10 | 28 |
| EBL | ECB | 5.4 ± 0.5 | 0 | 0 | 6 |
| | SCR | 0.8 ± 0.1* | 40 | 0 | 12 |
| CFL | ECB | 5.5 + 0.5 | 0 | 0 | 6 |
| | SCR | 0.2 ± 0.1* | 85 | 35 | 9 |
| MPL | ECB | 5.9 ±0.6 | 0 | 0 | 0 |
| | SCR | 0.2 ± 0.1* | 85 | 0 | 12 |
| RCA | ECB | 6.4 ± 0.5 | 0 | 0 | 6 |
| | SCR | 0.5 ± 0.1* | 65 | 10 | 9 |
| VVL | ECB | 6.4 ± 0.5 | 0 | 0 | 6 |
| | SCR | 0.5 ± 0.1* | 65 | 10 | 9 |

---

[1] Lectins were applied topically (2%) to Stoneville diet with larval weight and mortality recorded at seven days

[2] ECB=European corn borer and SCR = Southern corn rootworm.

[3] Weight is for surviving larve (Mean ± SEM, n=surviving observations). The weight of ECB control larvae for all

copical assays was 5.8+0.8 mg (Mean + SEM, n=102) with 5% mortality. Control SCR larvae weight was 1.4+0.1 mg (Mean + SEM, n=120) with 10% mortality.

[4] Percent weight loss was determined by dividing overall average of surviving larvae by overall mean of control larvae and multiplying by 100.

[5] Value has been adjusted for natural mortality and is based on number of insects, not observations.

[6] n=number of observations (usually 2 larvae observations[-1]).

[7] N.A.=Not applicable

[*]Value is significant from control at the p < 0.05 level

### Example 2

In another experiment, Stoneville media was prepared with only 90% of the original water. A 0.5% test composition was prepared using approximately 8.0 g of media and adding 45 mg of lectin in 1 mL PBS buffer. The media was then thoroughly mixed and poured into six cells. The addition of only 1 mL of buffer served as the control treatment. The cells were then infested and covered with mylar as described for the topical assays. Larval weights and mortalities were recorded at seven days and compared to the control treatment. If anti-insect activity was still observed at the 0.5% level, further tests were conducted at 0.3, 0.1, 0.05, and 0.01% levels of lectin incorporated in the dietary material. All percentages herein are by weight unless otherwise indicated.

### Results

Results are shown in Table 3. Bioassays of WGA, RCA and BPA incorporated in larval dietary materials at 5 mg lectin per gram of diet showed 100% larval mortality. WGA produced 80% mortality at a level of 1 mg/g, while RCA produced 90% mortality at the same level. BPA produced only 60% mortality at this level. The $LC_{50}$s were calculated to be 0.59 mg/g for WGA, 0.29 mg/g for RCA, and 0.73 mg/g for BPA.

WGA also inhibited weight gain in surviving ECB larvae. weight losses ranged form 90% near the $LC_{50}$ to 40% at the 0.1 mg/g level. RCA and BPA did not affect weight gain as dramatically as WGA. RCA inhibited growth by 40% near its $LC_{50}$, and BPA inhibited growth by 50% at its $LC_{50}$.

Nine lectins of the 46 tested had toxic effects on SCR larvae. These were RCA, CFL, VVL, GSL, WGA, jacalin, MPL, EBL and PAL. RCA had similar toxicity effects on SCR that were observed with ECB (Table 2). BPA failed to produce any mortality when screened for seven days against SCR. However, WGA, CFL, jacalin, VVL, MPL, EBL and GSL inhibited growth of larval SCR by 40% or greater. All seven lectins showed inhibition of weight gain that was significant at the p< 0.05 level. Larvae reared on diets containing CFL or MPL had the greatest reduction in growth of any treatment groups with an average weight of 0.2 mg. Larvae reared on diets containing jacalin, VVL, EBL and WGA had average weights of 0.4, 0.5, 0.6 and 0.6 mg, respectively. The control larvae had average weights of 1.4 mg ± 0.1 mg.

Table 3

| Effect of anti-insect lectins, incorporated into Stoneville diet, on growth and mortality of neonate European cornborer larvae[1]. | | | |
|---|---|---|---|
| Lectin | Concentration[2] | % Mortality | % Weight Loss |
| BPA lectin | 0.5%<br>0.1%<br>0.05%<br>0.01% | 90<br>25<br>13<br>0 | 99<br>65<br>35<br>10 |
| RCA | 0.1%<br>0.03%<br>0.01% | 90<br>40<br>10 | 75<br>40<br>10 |
| WGA | 0.5%<br>0.3%<br>0.1%<br>0.05%<br>0.03%<br>0.01% | 100<br>100<br>80<br>50<br>7<br>0 | N.A.<br>N.A.<br>99<br>90<br>90<br>45 |

[1] Weight and mortality were recorded at seven days.
[2] Concentration was percent of total weight of diet.

Approximately 1.5 g of diet was used per observations.

Industrial Applicability

I. Identification of the lectin gene and insertion into bacteria

In order to isolate the coding sequence for the lectin, it is necessary to have nucleotide sequence data which establishes an open reading frame (i.e., the correct triplet code for translation which should have only one "stop" signal at the very end of the gene.) It is also necessary to have an indication of where to look for the protease cleavage junction between the lectin and the replicase which precedes it in the sequence. This can be determined from the peptide sequence of the N-terminal portion of the lectin or by comparing the lectin sequence with that of other homologous lectins. This can generally be accomplished and the necessary information obtained without sequencing the entire gene. Once the sequence at both ends of the gene has been determined, the remainder of the gene can be cloned using restriction enzymes that flank the lectin coding region or, more preferably, by cloning the precise lectin coding region by oligonucleotide-directed amplification of DNA (polymerase chain reaction or PCR).

Once the gene has been isolated, it can be cloned into a bacterial expression vector with linkers added to create all three reading frames (using 8mer, 10mer, and 12mers each of which contain an ATG translational start site). The resulting vectors, containing the fragments of interest, can be inserted into, for example, BRL's Maximum Efficiency DH5 F′ IQ transformation competent E. coli cells. All three transformations, one for each linker, are then screened via minipreps for the presence and orientation of insert. Appropriate clones are then chosen to test for expression of the lectin gene.

Clones containing the properly oriented inserts are grown in culture medium conducive to the induction of the gene (LB medium with added IPTG). The cells are lysed and bacterial proteins are electrophoresed in SDS polyacrylamide gels and then transferred to nitrocellulose. The resulting protein blots are easily screened for presence of lectin using rabbit polyclonal and mouse monoclonal anti-lectin antibody.

Having determined the proper reading frame, it is then necessary to remove the gene from the bacterial expression vector. The linker at the start of the gene region supplies the necessary start codon.

II. Expression of the Lectin Gene in Plants

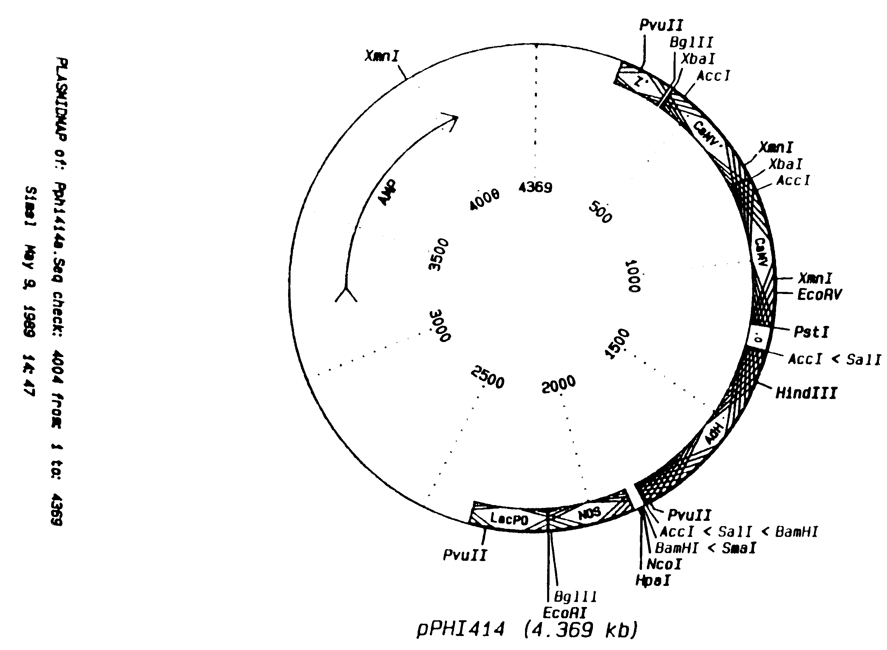

A plant expression cassette, employing the regulatory sequences developed by Beach, et al., and containing the lectin gene, is constructed. The restriction map of the preferred plasmid, designated pPHI414, is illustrated in Figure 1. This plasmid contains an enhanced 35S promoter spanning nucleotides -

11

EP 0 427 529 B1

421 to +2 of Cauliflower Mosaic Virus with the region from - 421 to - 90 duplicated in tandem, a 79 bp HindIII Sal1 fragment from pJII101 spanning the 5′ leader sequence of Tobacco Mosaic Virus, a 579 bp fragment spanning the first intron from maize AdH1-S, and a 281 bp fragment spanning the polyadenylation site from the nopaline synthase gene in pTiT37.

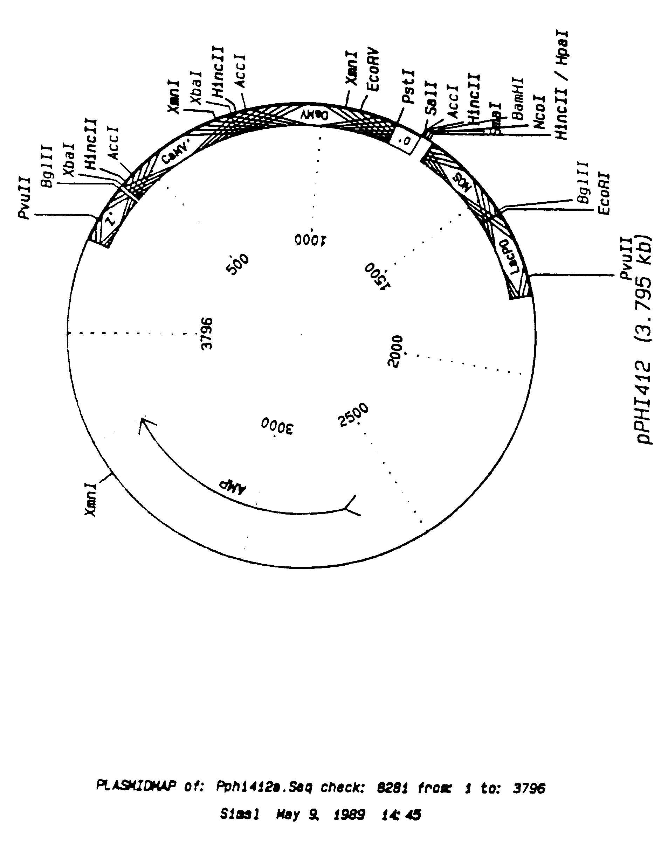

Another construct which can be used as an expression cassette is the pPHI412 plasmid shown in Figure 2. It differs from pPHI414 in that it lacks the AdH intron segment. However, like pPHI414, it is constructed to have numerous restriction sites between the O′ segment and the NOS segment, which sites can be conveniently used for splicing any desired lectin structural gene into position.

The latter point is illustrated specifically by plasmid pPHI224, a 5.029 kb plasmid which is based on pPHI414 and contains the structural gene which codes substantially solely for wheat germ agglutinin.

This vector can be cotransformed with a similar plasmid containing a selectable marker for antibiotic resistance into Black Mexican Sweet corn protoplasts by electroporation. These protoplasts can then be induced to regenerate cell walls and develop into callus by conventional techniques. Likewise, this callus can then be subjected to antibiotic selection to select for transformed colonies, and these colonies can be tested for expression of lectin with antisera for the appropriate lectin using known methods. The efficiency of protection can be measured by infesting callus (or suspension cultures derived from callus) with the target insect and measuring survival percentages.

The lectin gene can be introduced into embryogenic maize callus by methods similar to those used for Black Mexican Sweet. Embryogenic callus can be regenerated to whole fertile plants. The insect resistance imparted by the endogenous production of the lectin is a simply inherited, dominant trait and can, if desired, be introduced into other plant varieties of the species by simple crossing or backcrossing.

**Claims**
**Claims for the following Contracting States : AT, BE, CH, DE, DK, FR, GB, GR, IT, LI, LU, NL, SE**

1. A method for killing or inhibiting the growth of insect larvae selected from European corn borer, corn rootworm and cutworm, comprising administering orally to the larvae a larvicidal amount of a lectin selected from Artocarpus integrifolia lectin (jacalin), Bauhinia purpurea alba lectin, Codium fragile lectin, elderberry bark lectin, Griffonia simplicifolia lectin, Phytolacca americana lectin, Maclura pomifera lectin, Ricinus communis agglutinin, Wheat germ agglutinin and Vicia villosa lectin, and combinations thereof.

2. A method according to claim 1 wherein the lectin is administered orally by incorporating the lectin in the diet of the larvae.

3. A method according to claim 2 wherein the diet of the larvae is the tissues of a living plant.

4. A method according to claim 3 wherein the lectin comprises a lectin which is not native to the plant.

5. A method for protecting a plant against infestation by insect larvae selected from European corn borer, corn rootworm and cutworm, comprising inserting into the genome of the plant a sequence coding for at least one larvicidal plant lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Wheat germ agglutinin and Vicia villosa lectin, in proper reading frame relative to transcription initiator and promoter sequences active in the plant to cause expression of the lectin sequence at levels which provide a larvicidal amount of the lectin or combination of lectins in the tissues of the plant which are normally infested by the larvae.

6. A method according Claim 5 wherein the lectin or combination comprises a lectin which is not native to the plant.

7. A method according to Claim 5 wherein the lectin is wheat germ agglutinin.

8. A method according to Claim 5 wherein the lectin is Ricinus communis agglutinin.

9. A method according to Claim 5 wherein the plant is a monocotyledonous species selected from corn, wheat, rice and sorghum.

10. A method according to Claim 5 further comprising the steps of:

a) culturing cells or tissues from the plant,

b) introducing into the cells of the cell or tissue culture at least one copy of an expression cassette comprising a sequence coding for the lectin or combination of lectins, and

c) regenerating resistant whole plants from the cell or tissue culture.

**11.** A method according to Claim 10 which comprises the further step of sexually or clonally reproducing the whole plant in such manner that at least one copy of the sequence provided by the expression cassette is present in the cells of progeny of the reproduction.

**12.** A method according to Claim 10 in which the expression cassette is introduced into the cells by electroporation.

**13.** A method according to Claim 10 in which the expression cassette is introduced into the cells by microparticle bombardment.

**14.** A method according to Claim 10 in which the expression cassette is introduced into the cells by microinjection.

**15.** A method according to Claim 10 for providing resistance to insects in Agrobacterium tumefaciens-susceptible dicotyledonous plants in which the expression cassette is introduced into the cells by infecting the cells with Agrobacterium tumefaciens, a plasmid of which has been modified to include the expression cassette.

**16.** A method of imparting resistance to insects selected from European cornborer, corn rootworm, and cutworm to plants of a taxon susceptible to those insects, comprising the steps of:

a) selecting a fertile, insect resistant plant prepared by the method of Claim 10 from a sexually compatible plant;

b) sexually crossing the insect resistant plant with a plant from the insect susceptible taxon;

c) recovering reproductive material from the progeny of the cross; and

d) growing resistant plants from the reproductive material.

**17.** A method according to Claim 16 for imparting insect resistance in a taxon consisting of substantially homozygous plants, which comprises the further steps of repetitively:

a) backcrossing the insect resistant progeny with substantially homozygous, insect susceptible plants from the taxon; and

b) selecting for expression of both insect resistance and the other characteristics of the susceptible taxon among the progeny of the backcross, until the desired percentage of the characteristics of the susceptible taxon are present in the progeny along with insect resistance.

**18.** A DNA clone from the genome of a plant which codes substantially solely for at least one lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, wheat germ agglutinin, and Vicia villosa lectin.

**19.** An expression cassette comprising a DNA clone according to Claim 18 or a DNA clone from the genome of a plant which codes substantially solely for wheat germ agglutinin, operably linked to plant regulatory sequences which cause the expression of the DNA clone in plant cells.

**20.** An expression cassette comprising a DNA clone according to Claim 18 operably linked to bacterial expression regulatory sequences which cause the expression of the DNA clone in bacterial cells.

**21.** Bacterial cells containing as a foreign plasmid at least one copy of an expression cassette according to Claim 20.

**22.** Transformed plant cells containing as foreign DNA at least one copy of the DNA sequence of an expression cassette according to Claim 19.

EP 0 427 529 B1

23. Transformed cells according to Claim 22, further characterized in being cells of a monocotyledonous species.

24. Transformed cells according to Claim 23, further characterized in being maize, sorghum, wheat or rice cells.

25. Transformed cells according to Claim 22, further characterized in being cells of a dicotyledonous species.

26. Transformed cells according to Claim 25, further characterized in being soybean, alfalfa, tobacco or tomato cells.

27. A maize cell or tissue culture comprising cells according to claim 24.

28. A transformed maize plant, the cells of which contain as foreign DNA at least one copy of the DNA sequence of an expression cassette according to Claim 19.

29. A larvicidal composition, comprising a larvicidal amount of a plant lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Wheat germ agglutinin, and Vicia villosa lectin, and combinations thereof, in a non-phytotoxic vehicle.

30. A composition according to Claim 29 wherein the vehicle is adapted for systemic administration to a susceptible plant.

31. A composition according to Claim 30 wherein the vehicle further comprises a larval dietary bait for susceptible insects.

32. A composition according to Claim 31 wherein the bait further comprises a hormonal larval attractant for susceptible insects.

**Claims for the following Contracting State : ES**

1. A method for killing or inhibiting the growth of insect larvae selected from European corn borer, corn rootworm and cutworm, comprising administering orally to the larvae a larvicidal amount of a lectin selected from Artocarpus integrifolia lectin (jacalin), Bauhinia purpurea alba lectin, Codium fragile lectin, elderberry bark lectin, Griffonia simplicifolia lectin, Phytolacca americana lectin, Maclura pomifera lectin, Ricinus communis agglutinin, Wheat germ agglutinin and Vicia villosa lectin, and combinations thereof.

2. A method according to claim 1 wherein the lectin is administered orally by incorporating the lectin in the diet of the larvae.

3. A method according to claim 2 wherein the diet of the larvae is the tissues of a living plant.

4. A method according to claim 3 wherein the lectin comprises a lectin which is not native to the plant.

5. A method for protecting a plant against infestation by insect larvae selected from European corn borer, corn rootworm and cutworm, comprising inserting into the genome of the plant a sequence coding for at least one larvicidal plant lectin selected from jacalin, Bauhinia purpurea alba lectin, Codium fragile lectin, elderberry bark lectin, Griffonia simplicifolia lectin, Phytolacca americana lectin, Maclura pomifera lectin, Ricinus communis agglutinin, Wheat germ agglutinin and Vicia villosa lectin, in proper reading frame relative to transcription initiator and promoter sequences active in the plant to cause expression of the lectin sequence at levels which provide a larvicidal amount of the lectin or combination of lectins in the tissues of the plant which are normally infested by the larvae.

6. A method according Claim 5 wherein the lectin or combination comprises a lectin which is not native to the plant.

14

**7.** A method according to Claim 5 wherein the lectin is wheat germ agglutinin.

**8.** A method according to Claim 5 wherein the lectin is Ricinus communis agglutinin.

**9.** A method according to Claim 5 wherein the plant is a monocotyledonous species selected from corn, wheat, rice and sorghum.

**10.** A method according to Claim 5 further comprising the steps of:
a) culturing cells or tissues from the plant,
b) introducing into the cells of the cell or tissue culture at least one copy of an expression cassette comprising a sequence coding for the lectin or combination of lectins, and
c) regenerating resistant whole plants from the cell or tissue culture.

**11.** A method according to Claim 10 which comprises the further step of sexually or clonally reproducing the whole plant in such manner that at least one copy of the sequence provided by the expression cassette is present in the cells of progeny of the reproduction.

**12.** A method according to Claim 10 in which the expression cassette is introduced into the cells by electroporation.

**13.** A method according to Claim 10 in which the expression cassette is introduced into the cells by microparticle bombardment.

**14.** A method according to Claim 10 in which the expression cassette is introduced into the cells by microinjection.

**15.** A method according to Claim 10 for providing resistance to insects in Agrobacterium tumefaciens-susceptible dicotyledonous plants in which the expression cassette is introduced into the cells by infecting the cells with Agrobacterium tumefaciens, a plasmid of which has been modified to include the expression cassette.

**16.** A method of imparting resistance to insects selected from European cornborer, corn rootworm, and cutworm to plants of a taxon susceptible to those insects, comprising the steps of:
a) selecting a fertile, insect resistant plant prepared by the method of Claim 10 from a sexually compatible plant;
b) sexually crossing the insect resistant plant with a plant from the insect susceptible taxon;
c) recovering reproductive material from the progeny of the cross; and
d) growing resistant plants from the reproductive material.

**17.** A method according to Claim 16 for imparting insect resistance in a taxon consisting of substantially homozygous plants, which comprises the further steps of repetitively:
a) backcrossing the insect resistant progeny with substantially homozygous, insect susceptible plants from the taxon; and
b) selecting for expression of both insect resistance and the other characteristics of the susceptible taxon among the progeny of the backcross,until the desired percentage of the characteristics of the susceptible taxon are present in the progeny along with insect resistance.

**18.** A method of obtaining a DNA clone which codes for at least one larvicidal plant lectin selected from the lectins listed in claim 1 which comprises isolating DNA coding for the lectin or lectins from a plant or plant cells prepared in accordance with any one of claims 5 to 17.

**19.** A method as claimed in claim 18 wherein said DNA clone comprises an expression cassette including a coding sequence for a lectin listed in claim 1, preferably a coding sequence for wheat germ agglutinin operably linked to plant regulatory sequences capable of directing expression of said coding sequence in plant cells.

**20.** A method as claimed in claim 18 or claim 19 wherein said DNA clone comprises an expression cassette in which a coding sequence for a lectin listed in claim 1 is operably linked to bacterial expression regulatory sequences capable of directing expression of said coding sequence in bacterial cells.

21. A method of obtaining bacterial cells containing as a foreign plasmid at least one copy of an expression cassette in which a DNA coding sequence for a lectin listed in claim 1 is operably linked to bacterial expression regulatory sequences capable of directing expression of said coding sequence in said cells, said method comprising transforming said cells with said plasmid.

22. A method of obtaining plant cells containing as foreign DNA at least one copy of an expression cassette in which a DNA coding sequence for a lectin listed in claim 1 is operably linked to plant regulatory sequences capable of directing expression of said lectin in said plant cells, said method comprising transforming said plant cells with said foreign DNA.

23. A method as claimed in claim 22 wherein said plant cells are of a monocotyledonous species.

24. A method as claimed in claim 23 wherein said plant cells are selected from maize, sorghum, wheat and rice cells.

25. A method as claimed in claim 22 wherein said plant cells are of a dicotyledonous species.

26. A method as claimed in claim 25 wherein said plant cells are selected from soybean, alfalfa, tobacco and tomato cells.

27. A method as claimed in claim 24 where said plant cells are a maize cell culture.

28. A method as claimed in claim 27 wherein transformed maize cells are subsequently generated into transformed maize plants.

29. A method as claimed in claim 1 wherein said lectin is applied to plants susceptible to insect larvae selected from European corn borer, corn rootworm and cutworm as a larvicidal composition in combination with a non-phytotoxic vehicle.

30. A method as claimed in claim 29 wherein said larvicidal composition is adapted for systemic administration to a plant.

31. A method as claimed in claim 30 wherein said larvicidal composition further comprises a larval dietary bait for susceptible insect larvae.

32. A method as claimed in claim 31 wherein said bait comprises a hormonal larval attractant for susceptible insect larvae.


**Patentansprüche**
**Patentansprüche für folgende Vertragsstaaten : AT, BE, CH, DE, DK, FR, GB, GR, IT, LI, LU, NL, SE**

1. Verfahren zum Abtöten oder zum Verhindern des Wachstums von Insektenlarven, ausgewählt aus dem Maiszünsler, maiswurzelschädigenden Larven bzw. Nematoden (corn rootworm) und Eulenfalterraupen (cutworm), dadurch **gekennzeichnet,** daß man den Larven oral eine für die Larven tödliche Menge eines Lectins, ausgewählt aus Artocarpus-integrifolia-Lectin (Jacalin), Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin, sowie Kombinationen davon, verabreicht.

2. Verfahren nach Anspruch 1, dadurch **gekennzeichnet,** daß das Lectin oral verabreicht wird, indem man das Lectin in die Nahrung für die Larven einbringt.

3. Verfahren nach Anspruch 2, dadurch **gekennzeichnet,** daß die Nahrung für die Larven Gewebe einer lebenden Pflanze ist.

**4.** Verfahren nach Anspruch 3, dadurch **gekennzeichnet,** daß das Lectin ein Lectin umfaßt, welches nicht natürlich in der Pflanze vorkommt.

**5.** Verfahren zum Schutz einer Pflanze gegen den Befall durch Insektenlarven, ausgewählt aus dem Maiszünsler, maiswurzelschädigenden Larven bzw. Nematoden (corn rootworm) und Eulenfalterraupen (cutworm), dadurch **gekennzeichnet,** daß man in das Genom der Pflanze eine Sequenz, codierend für mindestens ein larvizides Pflanzenlectin, ausgewählt aus Jacalin, Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin, im, bezogen auf den Transkriptionsinitiator und die Promotorsequenzen, die in der Pflanze aktiv sind, richtigen Leseraster insertiert, um die Expression der Lectinsequenz in Konzentrationen zu bewirken, die eine für Larven tödliche Menge des Lectins oder der Kombination von Lectinen in den Geweben der Pflanze, die normalerweise durch die Larven befallen werden, bereitstellen.

**6.** Verfahren nach Anspruch 5, dadurch **gekennzeichnet,** daß das Lectin oder die Kombination ein Lectin umfaßt, welches nicht natürlich in der Pflanze vorkommt.

**7.** Verfahren nach Anspruch 5, dadurch **gekennzeichnet,** daß das Lectin Weizenkeim-Agglutinin ist.

**8.** Verfahren nach Anspruch 5, dadurch **gekennzeichnet,** daß das Lectin Ricinus-communis-Agglutinin ist.

**9.** Verfahren nach Anspruch 5, dadurch **gekennzeichnet,** daß die Pflanze eine einkeimblättrige Art, ausgewählt aus Mais, Weizen, Reis und Sorghumhirse ist.

**10.** Verfahren nach Anspruch 5, weiterhin **gekennzeichnet** durch die Stufen, daß man
(a) Zellen oder Gewebe der Pflanze kultiviert,
(b) in die Zellen der Zell- oder Gewebekultur mindestens eine Kopie einer Expressionskassette, umfassend eine Sequenz, die das Lectin oder die Lectinkombination codiert, einführt, und daß man
(c) resistente vollständige Pflanzen aus der Zell- oder Gewebekultur regeneriert.

**11.** Verfahren nach Anspruch 10, **gekennzeichnet** durch die weitere Stufe der geschlechtlichen oder über Clone erfolgenden Fortpflanzung der vollständigen Pflanze in der Weise, daß mindestens eine Kopie der durch die Expressionskassette bereitgestellten Sequenz in den Zellnachkommen der Fortpflanzung vorhanden ist.

**12.** Verfahren nach Anspruch 10, dadurch **gekennzeichnet,** daß die Expressionskassette in die Zellen durch Elektroporation eingeführt wird.

**13.** Verfahren nach Anspruch 10, dadurch **gekennzeichnet,** daß die Expressionskassette in die Zellen durch Mikroteilchenbeschuß eingeführt wird.

**14.** Verfahren nach Anspruch 10, dadurch **gekennzeichnet,** daß die Expressionskassette in die Zellen durch Mikroinjektion eingeführt wird.

**15.** Verfahren nach Anspruch 10, um für Agrobacterium tumefaciens anfällige zweikeimblättrige Pflanzen gegenüber Insekten resistent zu machen, dadurch **gekennzeichnet,** daß die Expressionskassette in die Zellen durch Infektion der Zellen mit Agrobacterium tumefaciens, von dem ein Plasmid so modifiziert worden ist, daß es die Expressionskassette enthält, eingeführt wird.

**16.** Verfahren, um Pflanzen Resistenz gegenüber Insekten, ausgewählt aus dem Maiszünsler, maiswurzelschädigenden Larven bzw. Nematoden (corn rootworm) und Eulenfalterraupen (cutworm) zu verleihen, wobei es sich um Pflanzen eines gegenüber diesen Insekten anfälligen Taxons handelt, **gekennzeichnet** durch die Stufen, daß man
(a) eine fertile, insektenresistente Pflanze, hergestellt aus einer sexuell kompatiblen Pflanze nach dem Verfahren von Anspruch 10, auswählt,
(b) die insektenresistente Pflanze mit einer Pflanze des gegen Insekten anfälligen Taxons geschlechtlich kreuzt,
(c) Fortpflanzungsmaterial aus den Nachkommen der Kreuzung gewinnt, und daß man

(d) aus dem Fortpflanzungsmaterial resistente Pflanzen zieht.

**17.** Verfahren nach Anspruch 16, um einem Taxon, das im wesentlichen aus homozygoten Pflanzen besteht, Insektenresistenz zu verleihen, **gekennzeichnet** durch die weiteren Stufen, daß man wiederholt

(a) die insektenresistenten Nachkommen mit im wesentlichen homozygoten, gegen Insekten anfälligen Pflanzen des Taxons rückkreuzt, und daß man

(b) bezüglich der Expression sowohl der Insektenresistenz als auch der anderen charakteristischen Eigenschaften des anfälligen Taxons unter den Nachkommen der Rückkreuzung selektioniert, bis der gewünschte Prozentsatz der charakteristischen Eigenschaften des anfälligen Taxons zusammen mit der Insektenresistenz in den Nachkommen vorliegt.

**18.** DNA-Clon aus dem Genom einer Pflanze, der im wesentlichen nur für mindestens ein Lectin, ausgewählt aus Jacalin, Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin, codiert.

**19.** Expressionskassette, umfassend einen DNA-Clon nach Anspruch 18 oder einen DNA-Clon aus dem Genom einer Pflanze, der im wesentlichen nur Weizenkeim-Agglutinin codiert, der funktionell mit Pflanzenregulationssequenzen verbunden ist, welche die Expression des DNA-Clons in Pflanzenzellen bewirken.

**20.** Expressionskassette, umfassend einen DNA-Clon nach Anspruch 18, der funktionell mit Regulationssequenzen der bakteriellen Expression verbunden ist, die die Expression des DNA-Clons in Bakterienzellen bewirken.

**21.** Bakterienzellen, die als Fremd-Plasmid mindestens eine Kopie einer Expressionskassette nach Anspruch 20 enthalten.

**22.** Transformierte Pflanzenzellen, die als Fremd-DNA mindestens eine Kopie der DNA-Sequenz einer Expressionskassette nach Anspruch 19 enthalten.

**23.** Transformierte Zellen nach Anspruch 22, weiterhin dadurch **gekennzeichnet,** daß sie Zellen einer einkeimblättrigen Art sind.

**24.** Transformierte Zellen nach Anspruch 23, weiterhin dadurch **gekennzeichnet,** daß sie Mais-, Sorghumhirse-, Weizen- oder Reiszellen sind.

**25.** Transformierte Zellen nach Anspruch 22, weiterhin dadurch **gekennzeichnet,** daß sie Zellen einer zweikeimblättrigen Art sind.

**26.** Transformierte Zellen nach Anspruch 25, weiterhin dadurch **gekennzeichnet,** daß sie Sojabohnen-, Luzerne- (Alfalfa), Tabak- oder Tomatenzellen sind.

**27.** Maiszell- oder Maisgewebekultur, umfassend Zellen nach Anspruch 24.

**28.** Transformierte Maispflanze, deren Zellen als Fremd-DNA mindestens eine Kopie der DNA-Sequenz einer Expressionskassette nach Anspruch 19 enthalten.

**29.** Larvizides Mittel, umfassend eine für Larven tödliche Menge eines Pflanzenlectins, ausgewählt aus Jacalin, Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin und Kombinationen davon, in einem nicht-phytotoxischen Träger.

**30.** Mittel nach Anspruch 29, dadurch **gekennzeichnet,** daß der Träger für die systemische Verabreichung an eine anfällige Pflanze angepaßt ist.

**31.** Mittel nach Anspruch 30, dadurch **gekennzeichnet,** daß der Träger weiterhin einen Larvennahrungsköder für anfällige Insekten umfaßt.

**32.** Mittel nach Anspruch 31, dadurch **gekennzeichnet,** daß der Köder weiterhin einen Larvenhormonlockstoff für anfällige Insekten umfaßt.

**Patentansprüche für folgenden Vertragsstaat : ES**

**1.** Verfahren zum Abtöten oder zum Verhindern des Wachstums von Insektenlarven, ausgewählt aus dem Maiszünsler, maiswurzelschädigenden Larven bzw. Nematoden (corn rootworm) und Eulenfalterraupen (cutworm), dadurch **gekennzeichnet,** daß man den Larven oral eine für die Larven tödliche Menge eines Lectins, ausgewählt aus Artocarpus-integrifolia-Lectin (Jacalin), Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin, sowie Kombinationen davon, verabreicht.

**2.** Verfahren nach Anspruch 1, dadurch **gekennzeichnet,** daß das Lectin oral verabreicht wird, indem man das Lectin in die Nahrung für die Larven einbringt.

**3.** Verfahren nach Anspruch 2, dadurch **gekennzeichnet,** daß die Nahrung für die Larven Gewebe einer lebenden Pflanze ist.

**4.** Verfahren nach Anspruch 3, dadurch **gekennzeichnet,** daß das Lectin ein Lectin umfaßt, welches nicht natürlich in der Pflanze vorkommt.

**5.** Verfahren zum Schutz einer Pflanze gegen den Befall durch Insektenlarven, ausgewählt aus dem Maiszünsler, maiswurzelschädigenden Larven bzw. Nematoden (corn rootworm) und Eulenfalterraupen (cutworm), dadurch **gekennzeichnet,** daß man in das Genom der Pflanze eine Sequenz, codierend für mindestens ein larvizides Pflanzenlectin, ausgewählt aus Jacalin, Bauhinia-purpurea-alba-Lectin, Codium-fragile-Lectin, Holunderrinden-Lectin, Griffonia-simplicifolia-Lectin, Phytolacca-americana-Lectin, Maclura-pomifera-Lectin, Ricinus-communis-Agglutinin, Weizenkeim-Agglutinin und Vicia-villosa-Lectin, im, bezogen auf den Transkriptionsinitiator und die Promotorsequenzen, die in der Pflanze aktiv sind, richtigen Leseraster insertiert, um die Expression der Lectinsequenz in Konzentrationen zu bewirken, die eine für Larven tödliche Menge des Lectins oder der Kombination von Lectinen in den Geweben der Pflanze, die normalerweise durch die Larven befallen werden, bereitstellen.

**6.** Verfahren nach Anspruch 5, dadurch **gekennzeichnet,** daß das Lectin oder die Kombination ein Lectin umfaßt, welches nicht natürlich in der Pflanze vorkommt.

**7.** Verfahren nach Anspruch 5, dadurch **gekennzeichnet,** daß das Lectin Weizenkeim-Agglutinin ist.

**8.** Verfahren nach Anspruch 5, dadurch **gekennzeichnet,** daß das Lectin Ricinus-communis-Agglutinin ist.

**9.** Verfahren nach Anspruch 5, dadurch **gekennzeichnet,** daß die Pflanze eine einkeimblättrige Art, ausgewählt aus Mais, Weizen, Reis und Sorghumhirse ist.

**10.** Verfahren nach Anspruch 5, weiterhin **gekennzeichnet** durch die Stufen, daß man
(a) Zellen oder Gewebe der Pflanze kultiviert,
(b) in die Zellen der Zell- oder Gewebekultur mindestens eine Kopie einer Expressionskassette, umfassend eine Sequenz, die das Lectin oder die Lectinkombination codiert, einführt, und daß man
(c) resistente vollständige Pflanzen aus der Zell- oder Gewebekultur regeneriert.

**11.** Verfahren nach Anspruch 10, **gekennzeichnet** durch die weitere Stufe der geschlechtlichen oder über Clone erfolgenden Fortpflanzung der vollständigen Pflanze in der Weise, daß mindestens eine Kopie der durch die Expressionskassette bereitgestellten Sequenz in den Zellnachkommen der Fortpflanzung vorhanden ist.

**12.** Verfahren nach Anspruch 10, dadurch **gekennzeichnet,** daß die Expressionskassette in die Zellen durch Elektroporation eingeführt wird.

**13.** Verfahren nach Anspruch 10, dadurch **gekennzeichnet,** daß die Expressionskassette in die Zellen durch Mikroteilchenbeschuß eingeführt wird.

**14.** Verfahren nach Anspruch 10, dadurch **gekennzeichnet,** daß die Expressionskassette in die Zellen durch Mikroinjektion eingeführt wird.

**15.** Verfahren nach Anspruch 10, um für Agrobacterium tumefaciens anfällige zweikeimblättrige Pflanzen gegenüber Insekten resistent zu machen, dadurch **gekennzeichnet,** daß die Expressionskassette in die Zellen durch Infektion der Zellen mit Agrobacterium tumefaciens, von dem ein Plasmid so modifiziert worden ist, daß es die Expressionskassette enthält, eingeführt wird.

**16.** Verfahren, um Pflanzen Resistenz gegenüber Insekten, ausgewählt aus dem Maiszünsler, maiswurzelschädigenden Larven bzw. Nematoden (corn rootworm) und Eulenfalterraupen (cutworm) zu verleihen, wobei es sich um Pflanzen eines gegenüber diesen Insekten anfälligen Taxons handelt, **gekennzeichnet** durch die Stufen, daß man
(a) eine fertile, insektenresistente Pflanze, hergestellt aus einer sexuell kompatiblen Pflanze nach dem Verfahren von Anspruch 10, auswählt,
(b) die insektenresistente Pflanze mit einer Pflanze des gegen Insekten anfälligen Taxons geschlechtlich kreuzt,
(c) Fortpflanzungsmaterial aus den Nachkommen der Kreuzung gewinnt, und daß man
(d) aus dem Fortpflanzungsmaterial resistente Pflanzen zieht.

**17.** Verfahren nach Anspruch 16, um einem Taxon, das im wesentlichen aus homozygoten Pflanzen besteht, Insektenresistenz zu verleihen, **gekennzeichnet** durch die weiteren Stufen, daß man wiederholt
(a) die insektenresistenten Nachkommen mit im wesentlichen homozygoten, gegen Insekten anfälligen Pflanzen des Taxons rückkreuzt, und daß man
(b) bezüglich der Expression sowohl der Insektenresistenz als auch der anderen charakteristischen Eigenschaften des anfälligen Taxons unter den Nachkommen der Rückkreuzung selektioniert, bis der gewünschte Prozentsatz der charakteristischen Eigenschaften des anfälligen Taxons zusammen mit der Insektenresistenz in den Nachkommen vorliegt.

**18.** Verfahren, um einen DNA-Clon zu erhalten, der mindestens ein larvizides Pflanzen-Lectin, ausgewählt aus den in Anspruch 1 aufgezählten Lectinen, codiert, welches umfaßt, daß man die für das Lectin oder die Lectine codierende DNA aus einer Pflanze oder aus Pflanzenzellen, hergestellt nach einem der Ansprüche 5 bis 17, isoliert.

**19.** Verfahren nach Anspruch 18, dadurch **gekennzeichnet,** daß der DNA-Clon eine Expressionskassette umfaßt, die eine codierende Sequenz für ein in Anspruch 1 aufgezähltes Lectin, bevorzugt eine codierende Sequenz für Weizenkeim-Agglutinin, funktionell verbunden mit Pflanzenregulationssequenzen, die in der Lage sind, die Expression der codierenden Sequenz in Pflanzenzellen zu steuern, enthält.

**20.** Verfahren nach Anspruch 18 oder 19, dadurch **gekennzeichnet,** daß der DNA-Clon eine Expressionskassette umfaßt, in der eine codierende Sequenz für ein in Anspruch 1 aufgezähltes Lectin funktionell mit Regulationssequenzen für die bakterielle Expression, die in der Lage sind, die Expression der codierenden Sequenz in Bakterienzellen zu steuern, verbunden ist.

**21.** Verfahren, um Bakterienzellen zu erhalten, die als Fremd-Plasmid mindestens eine Kopie einer Expressionskassette enthalten, in der eine codierende DNA-Sequenz für ein in Anspruch 1 aufgelistetes Lectin funktionell mit Regulationssequenzen der bakteriellen Expression verbunden ist, die in der Lage sind, die Expression der codierenden Sequenz in den Zellen zu steuern, wobei das Verfahren das Transformieren der Zellen mit dem Plasmid umfaßt.

**22.** Verfahren, um Pflanzenzellen zu erhalten, die als Fremd-DNA mindestens eine Kopie einer Expressionskassette enthalten, in der eine codierende DNA-Sequenz für ein in Anspruch 1 aufgezähltes Lectin funktionell mit Pflanzenregulationssequenzen verknüpft ist, die in der Lage sind, die Expression des Lectins in den Pflanzenzellen zu regeln, wobei das Verfahren das Transformieren der Pflanzenzellen mit der Fremd-DNA umfaßt.

**23.** Verfahren nach Anspruch 22, dadurch **gekennzeichnet,** daß die Pflanzenzellen von einer einkeimblättrigen Art stammen.

**24.** Verfahren nach Anspruch 23, dadurch **gekennzeichnet,** daß die Pflanzenzellen aus Mais-, Sorghumhirse-, Weizen- und Reiszellen ausgewählt werden.

**25.** Verfahren nach Anspruch 22, dadurch **gekennzeichnet,** daß die Pflanzenzellen von einer zweikeimblättrigen Art stammen.

**26.** Verfahren nach Anspruch 25, dadurch **gekennzeichnet,** daß die Pflanzenzellen aus Sojabohnen-, Luzerne- (Alfalfa), Tabak- und Tomatenzellen ausgewählt werden.

**27.** Verfahren nach Anspruch 24, dadurch **gekennzeichnet,** daß die Pflanzenzellen eine Maiszellkultur sind.

**28.** Verfahren nach Anspruch 27, dadurch **gekennzeichnet,** daß aus den transformierten Maiszellen anschließend transformierte Maispflanzen erzeugt werden.

**29.** Verfahren nach Anspruch 1, dadurch **gekennzeichnet,** daß das Lectin auf Pflanzen, die gegen Insektenlarven, ausgewählt aus dem Maiszünsler, maiswurzelschädigenden Larven bzw. Nematoden (corn rootworm) und Eulenfalterraupen (cutworm), anfällig sind, als larvizides Mittel in Kombination mit einem nicht-phytotoxischen Träger appliziert wird.

**30.** Verfahren nach Anspruch 29, dadurch **gekennzeichnet,** daß das larvizide Mittel für die systemische Verabreichung an Pflanzen angepaßt ist.

**31.** Verfahren nach Anspruch 30, dadurch **gekennzeichnet,** daß das larvizide Mittel weiterhin einen Larvennahrungsköder für anfällige Insektenlarven umfaßt.

**32.** Verfahren nach Anspruch 31, dadurch **gekennzeichnet,** daß der Köder einen Larvenhormonlockstoff für anfällige Insektenlarven umfaßt.

**Revendications**

**Revendications pour les Etats contractants suivants : AT, BE, CH, DE, DK, FR, GB, GR, IT, LI, LU, NL, SE**

**1.** Procédé pour tuer ou inhiber la croissance des larves d'insectes choisis parmi l'insecte térébrant du maïs Européen, le vers des racines du maïs et l'agrotis des moissons, comprenant l'administration, par voie orale, aux larves d'une quantité larvicide d'une lectine choisie parmi la lectine d'Artocarpus integrifolia (jacaline), la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé, la lectine de Vicia villosa, et leurs combinaisons.

**2.** Procédé selon la revendication 1, dans lequel la lectine est administrée par voie orale, par incorporation de la lectine à la nourriture des larves.

**3.** Procédé selon la revendication 2, dans lequel la nourriture des larves est constituée de tissus d'une plante vivante.

**4.** Procédé selon la revendication 3, dans lequel la lectine comprend une lectine qui n'est pas originaire de la plante.

5. Procédé pour protéger une plante contre l'infestation par des larves d'insectes choisis parmi l'insecte térébrant du maïs Européen, le vers des racines du maïs et l'agrotis des moissons, comprenant l'insertion, dans le génome de la plante, d'une séquence codant pour au moins une lectine de plante larvicide choisie parmi la jacaline, la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé et la lectine de Vicia villosa, dans un cadre de lecture approprié par rapport aux séquences promotrice et initiatrice de la transcription qui sont actives dans la plante, afin d'induire l'expression de la séquence de la lectine à des taux qui procurent une quantité larvicide de la lectine ou d'une combinaison de lectines dans les tissus de la plante qui sont normalement infestés par les larves.

6. Procédé selon la revendication 5, dans lequel la lectine ou la combinaison comprend une lectine qui n'est pas originaire de la plante.

7. Procédé selon la revendication 5, dans lequel la lectine est l'agglutinine de germe de blé.

8. Procédé selon la revendication 5, dans lequel la lectine est l'agglutinine de Ricinus communis.

9. Procédé selon la revendication 5, dans lequel la plante est une espèce monocotylédone choisie parmi le maïs, le blé, le riz et le sorgho.

10. Procédé selon la revendication 5, comprenant en outre les étapes consistant à :
    a) cultiver les cellules ou tissus de la plante,
    b) introduire dans les cellules de la culture cellulaire ou tissulaire au moins une copie d'une cassette d'expression comprenant une séquence codant pour la lectine ou une combinaison de lectines, et
    c) régénérer des plantes entières résistantes à partir de la culture cellulaire ou tissulaire.

11. Procédé selon la revendication 10, qui comprend l'étape supplémentaire de reproduction sexuée ou par clonage de la plante entière, de manière telle qu'au moins une copie de la séquence fournie par la cassette d'expression soit présente dans les cellules des descendants issus de la reproduction.

12. Procédé selon la revendication 10, dans lequel la cassette d'expression est introduite dans les cellules par électroporation.

13. Procédé selon la revendication 10, dans lequel la cassette d'expression est introduite dans les cellules par bombardement de microparticules.

14. Procédé selon la revendication 10, dans lequel la cassette d'expression est introduite dans les cellules par microinjection.

15. Procédé selon la revendication 10, destiné à rendre résistantes aux insectes des plantes dicotylédones sensibles à Agrobacterium tumefaciens, dans lequel la cassette d'expression est introduite dans les cellules en infectant les cellules avec Agrobacterium tumefaciens, dont un plasmide a été modifié pour y inclure la cassette d'expression.

16. Procédé destiné à rendre résistantes à des insectes choisis parmi l'insecte térébrant du maïs Européen, le vers des racines du maïs et l'agrotis des moissons, des plantes d'un taxon sensible à ces insectes, comprenant les étapes consistant à :
    a) sélectionner une plante résistante aux insectes, fertile, préparée selon le procédé de la revendication 10, à partir d'une plante sexuellement compatible;
    b) croiser sexuellement la plante résistante aux insectes avec une plante provenant du taxon sensible aux insectes;
    c) prélever du matériel reproducteur provenant des descendants du croisement; et
    d) faire croître des plantes résistantes à partir du matériel reproducteur.

17. Procédé selon la revendication 16, destiné à rendre résistant aux insectes un taxon constitué par des plantes essentiellement homozygotes, qui comprend les étapes répétitives supplémentaires, consistant à :

a) croiser en retour les descendants résistants aux insectes avec les plantes essentiellement homozygotes, sensibles aux insectes, du taxon; et

b) sélectionner, parmi les descendants du croisement en retour, pour l'expression à la fois de la résistance aux insectes ainsi que d'autres caractéristiques du taxon sensible, jusqu'à obtention chez les descendants du pourcentage souhaité de caractéristiques du taxon sensible, associé à la résistance aux insectes.

18. Clone d'ADN, provenant du génome d'une plante, qui code essentiellement seulement pour au moins une lectine choisie parmi la jacaline, la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé et la lectine de Vicia villosa.

19. Cassette d'expression comprenant un clone d'ADN selon la revendication 18, ou un clone d'ADN provenant du génome d'une plante, qui code essentiellement seulement pour l'agglutinine de germe de blé, lié de manière fonctionnelle à des séquences régulatrices de plante qui induisent l'expression du clone d'ADN dans des cellules de plante.

20. Cassette d'expression comprenant un clone d'ADN selon la revendication 18, lié de manière fonctionnelle à des séquences régulatrices d'expression bactérienne qui induisent l'expression du clone d'ADN dans des cellules bactériennes.

21. Cellules bactériennes contenant, en tant que plasmide étranger, au moins une copie d'une cassette d'expression selon la revendication 20.

22. Cellules de plante transformées contenant, en tant qu'ADN étranger, au moins une copie de la séquence d'ADN d'une cassette d'expression selon la revendication 19.

23. Cellules transformées selon la revendication 22, caractérisées en outre en ce que ce sont des cellules d'une espèce monocotylédone.

24. Cellules transformées selon la revendication 23, caractérisées en outre en ce que ce sont des cellules de maïs, de sorgho, de blé ou de riz.

25. Cellules transformées selon la revendication 22, caractérisées en outre en ce que ce sont des cellules d'une espèce dicotylédone.

26. Cellules transformées selon la revendication 25, caractérisées en outre en ce que ce sont des cellules de soja, de luzerne, de tabac ou de tomate.

27. Culture de cellules ou de tissu de maïs, comprenant des cellules selon la revendication 24.

28. Plante de maïs transformée, dont les cellules contiennent, en tant qu'ADN étranger, au moins une copie de la séquence d'ADN d'une cassette d'expression selon la revendication 19.

29. Composition larvicide, comprenant une quantité larvicide d'une lectine de plante choisie parmi la jacaline, la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé, la lectine de Vicia villosa, et leurs combinaisons, dans un véhicule non phytotoxique.

30. Composition selon la revendication 29, dans laquelle le véhicule est adapté à une administration systémique à une plante sensible.

31. Composition selon la revendication 30, dans laquelle le véhicule comprend, en outre, un appât sous forme de nourriture pour larve pour des insectes sensibles.

EP 0 427 529 B1

**32.** Composition selon la revendication 31, dans laquelle l'appât comprend, en outre, un produit hormonal qui attire les larves pour des insectes sensibles.

**Revendications pour l'Etat contractant suivant : ES**

**1.** Procédé pour tuer ou inhiber la croissance des larves d'insectes choisis parmi l'insecte térébrant du maïs Européen, le vers des racines du maïs et l'agrotis des moissons, comprenant l'administration, par voie orale, aux larves d'une quantité larvicide de lectine choisie parmi la lectine d'Artocarpus integrifolia (jacaline), la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé, la lectine de Vicia villosa, et leurs combinaisons.

**2.** Procédé selon la revendication 1, dans lequel la lectine est administrée par voie orale, par incorporation de la lectine à la nourriture des larves.

**3.** Procédé selon la revendication 2, dans lequel la nourriture des larves est constituée de tissus d'une plante vivante.

**4.** Procédé selon la revendication 3, dans lequel la lectine comprend une lectine qui n'est pas originaire de la plante.

**5.** Procédé pour protéger une plante contre l'infestation par des larves d'insectes choisis parmi l'insecte térébrant du maïs Européen, le vers des racines du maïs et l'agrotis des moissons, comprenant l'insertion, dans le génome de la plante, d'une séquence codant pour au moins une lectine de plante larvicide choisie parmi la jacaline, la lectine de Bauhinia purpurea alba, la lectine de Codium fragile, la lectine d'écorce de sureau, la lectine de Griffonia simplicifolia, la lectine de Phytolacca americana, la lectine de Maclura pomifera, l'agglutinine de Ricinus communis, l'agglutinine de germe de blé et la lectine de Vicia villosa, dans un cadre de lecture approprié par rapport aux séquences promotrice et initiatrice de la transcription qui sont actives dans la plante, afin d'induire l'expression de la séquence de la lectine à des taux qui procurent une quantité larvicide de la lectine ou d'une combinaison de lectines dans les tissus de la plante qui sont normalement infestés par les larves.

**6.** Procédé selon la revendication 5, dans lequel la lectine ou la combinaison comprend une lectine qui n'est pas originaire de la plante.

**7.** Procédé selon la revendication 5, dans lequel la lectine est l'agglutinine de germe de blé.

**8.** Procédé selon la revendication 5, dans lequel la lectine est l'agglutinine de Ricinus communis.

**9.** Procédé selon la revendication 5, dans lequel la plante est une espèce monocotylédone choisie parmi le maïs, le blé, le riz et le sorgho.

**10.** Procédé selon la revendication 5, comprenant en outre les étapes consistant à :
   a) cultiver les cellules ou tissus de la plante,
   b) introduire dans les cellules de la culture cellulaire ou tissulaire au moins une copie d'une cassette d'expression comprenant une séquence codant pour la lectine ou une combinaison de lectines, et
   c) régénérer des plantes entières résistantes à partir de la culture cellulaire ou tissulaire.

**11.** Procédé selon la revendication 10, qui comprend l'étape supplémentaire de reproduction sexuée ou par clonage de la plante entière, de manière telle qu'au moins une copie de la séquence fournie par la cassette d'expression soit présente dans les cellules des descendants issus de la reproduction.

**12.** Procédé selon la revendication 10, dans lequel la cassette d'expression est introduite dans les cellules par électroporation.

**13.** Procédé selon la revendication 10, dans lequel la cassette d'expression est introduite dans les cellules par bombardement de microparticules.

24

**14.** Procédé selon la revendication 10, dans lequel la cassette d'expression est introduite dans les cellules par microinjection.

**15.** Procédé selon la revendication 10, destiné à rendre résistantes aux insectes des plantes dicotylédones sensibles à Agrobacterium tumefaciens, dans lequel la cassette d'expression est introduite dans les cellules en infectant les cellules avec Agrobacterium tumefaciens, dont un plasmide a été modifié pour y inclure la cassette d'expression.

**16.** Procédé destiné à rendre résistantes à des insectes choisis parmi l'insecte térébrant du maïs Européen, le vers des racines du maïs et l'agrotis des moissons, des plantes d'un taxon sensible à ces insectes, comprenant les étapes consistant à :

a) sélectionner une plante résistante aux insectes, fertile, préparée selon le procédé de la revendication 10, à partir d'une plante sexuellement compatible;

b) croiser sexuellement la plante résistante aux insectes avec une plante provenant du taxon sensible aux insectes;

c) prélever du matériel reproducteur provenant des descendants du croisement; et

d) faire croître des plantes résistantes à partir du matériel reproducteur.

**17.** Procédé selon la revendication 16, destiné à rendre résistant aux insectes un taxon constitué par des plantes essentiellement homozygotes, qui comprend les étapes répétitives supplémentaires, consistant à :

a) croiser en retour les descendants résistants aux insectes avec les plantes essentiellement homozygotes, sensibles aux insectes, du taxon; et

b) sélectionner, parmi les descendants du croisement en retour, pour l'expression à la fois de la résistance aux insectes ainsi que d'autres caractéristiques du taxon sensible, jusqu'à obtention chez les descendants du pourcentage souhaité de caractéristiques du taxon sensible, associé à la résistance aux insectes.

**18.** Procédé d'obtention d'un clone d'ADN qui code pour au moins une lectine de plante larvicide choisie parmi les lectines énumérées dans la revendication 1, qui comprend l'isolement de l'ADN codant pour la lectine ou les lectines à partir d'une plante ou de cellules de plante préparées selon l'une quelconque des revendications 5 à 17.

**19.** Procédé tel que revendiqué dans la revendication 18, dans lequel ledit clone d'ADN comprend une cassette d'expression incluant une séquence codant pour une lectine citée dans la revendication 1, de préférence une séquence codant pour l'agglutinine de germe de blé, liée de manière fonctionnelle à des séquences régulatrices de plante capables de diriger l'expression de ladite séquence codante dans des cellules de plante.

**20.** Procédé tel que revendiqué dans la revendication 18 ou la revendication 19, dans lequel ledit clone d'ADN comprend une cassette d'expression dans laquelle une séquence codant pour une lectine citée dans la revendication 1 est liée de manière fonctionnelle à des séquences régulatrices d'expression bactériennes, capables de diriger l'expression de ladite séquence codante dans des cellules bactériennes.

**21.** Procédé d'obtention de cellules bactériennes contenant, en tant que plasmide étranger, au moins une copie d'une cassette d'expression dans laquelle une séquence d'ADN codant pour une lectine citée dans la revendication 1 est liée de manière fonctionnelle à des séquences régulatrices d'expression bactériennes, capables de diriger l'expression de ladite séquence codante dans lesdites cellules, ledit procédé comprenant la transformation desdites cellules avec ledit plasmide.

**22.** Procédé d'obtention de cellules de plante contenant, en tant qu'ADN étranger, au moins une copie d'une cassette d'expression dans laquelle une séquence d'ADN codant pour une lectine mentionnée dans la revendication 1 est liée de manière fonctionnelle à des séquences régulatrices de plante capables de diriger l'expression de ladite lectine dans lesdites cellules de plante, ledit procédé comprenant la transformation desdites cellules de plante avec ledit ADN étranger.

**23.** Procédé tel que revendiqué dans la revendication 22, dans lequel lesdites cellules de plante appartiennent à une espèce monocotylédone.

**24.** Procédé tel que revendiqué dans la revendication 23, dans lequel lesdites cellules de plante sont choisies parmi les cellules de maïs, de sorgho, de blé ou de riz.

**25.** Procédé tel que revendiqué dans la revendication 22, dans lequel lesdites cellules de plante sont d'une espèce dicotylédone.

**26.** Procédé tel que revendiqué dans la revendication 25, dans lequel lesdites cellules de plante sont choisies parmi les cellules de soja, de luzerne, de tabac ou de tomate.

**27.** Procédé tel que revendiqué dans le revendication 24, dans lequel lesdites cellules de plante sont une culture de cellules de maïs.

**28.** Procédé tel que revendiqué dans la revendication 27, dans lequel les cellules de maïs transformées génèrent par la suite des plantes de maïs transformées.

**29.** Procédé tel que revendiqué dans la revendication 1, dans lequel ladite lectine est administrée à des plantes sensibles aux larves d'insectes choisis parmi l'insecte térébrant du maïs Européen, le vers des racines du maïs et l'agrotis des moissons, sous forme d'une composition larvicide en combinaison avec un véhicule non phytotoxique.

**30.** Procédé tel que revendiqué dans la revendication 29, dans lequel ladite composition larvicide est adaptée à une administration systémique à une plante.

**31.** Procédé tel que revendiqué dans la revendication 30, dans lequel ladite composition larvicide comprend, en outre, un appât sous forme de nourriture pour larve destiné aux larves d'insectes sensibles.

**32.** Procédé tel que revendiqué dans la revendication 31, dans lequel ledit l'appât comprend un produit hormonal, qui attire les larves, destiné aux larves d'insectes sensibles.

PLASMIDMAP of: Pph1414a.Seq check: 4004 from 1 to: 4369

SimaI May 9, 1989 14:47

pPHI412 (3.795 kb)

PLASMIDMAP of: Pphi412a.Seq check: 8281 from 1 to: 3796

Sims1 May 9, 1989 14:45

28

pPHI 224 (5.029kb)

PLASMIDMAP of: pphi224 Check: 6000 From: 1 TO: 5029

Fall: September 7, 1969 09:29

29